# EUROPEAN PATENT SPECIFICATION

(11) **EP 2 591 362 B1**
(45) Date of publication and mention of the grant of the patent: **25.02.2015**
(21) Application number: 11754530.1
(22) Date of filing: 08.07.2011
(51) Int. Cl.: G01N 33/574

(54) **USE OF MELANOMA BIOMARKERS IN MEDICAL AND DIAGNOSTIC FIELD AND METHOD FOR THE IDENTIFICATION THEREOF**
VERWENDUNG VON MELANOMBIOMARKERN FÜR MEDIZINISCHE UND DIAGNOSTISCHE ZWECKE SOWIE VERFAHREN FÜR IHRE DIAGNOSTIZIERUNG
UTILISATION DE BIOMARQUEURS DE MÉLANOME DANS LE DOMAINE MÉDICAL ET DU DIAGNOSTIC ET PROCÉDÉ POUR LEUR IDENTIFICATION

(30) Priority: 30.09.2010 IT RM20100505; 09.07.2010 IT RM20100377
(43) Date of publication of application: 15.05.2013
(73) Proprietor: Istituto Superiore di Sanità, 00161 Roma (IT)
(72) Inventor: FACCHIANO, Francesco, I-00161 Roma (IT)
(74) Representative: Gitto, Serena
(86) International application number: PCT/IT2011/000234
(87) International publication number: WO 2012/004822

(56) References cited:
- JEROME SOLASSOL ET AL: "Identification of serum melanoma progression biomarkers through proteomic-based approaches", EXPERT REVIEW OF PROTEOMICS, vol. 6, no. 4, 1 August 2009 (2009-08-01), pages 341-343, XP055010487, ISSN: 1478-9450, DOI: 10.1586/epr.09.57
- SILVER H K ET AL: "Relationship of total serum sialic acid to sialylglycoprotein acute-phase reactants in malignant melanoma", BRITISH JOURNAL OF CANCER, NATURE PUBLISHING GROUP, LONDON, GB, vol. 41, no. 5, 1 May 1980 (1980-05-01), pages 745-750, XP008096543, ISSN: 0007-0920
- WOJTUKIEWICZ M Z ET AL: "Profiles of plasma serpins in patients with advanced malignant melanoma, gastric cancer and breast cancer", HAEMOSTASIS, vol. 28, no. 1, January 1998 (1998-01), pages 7-13, XP008144611, ISSN: 0301-0147
- KWAAN H C ET AL: "TISSUE PLASMINOGEN ACTIVATOR AND INHIBITORS OF FIBRINOLYSIS IN MALIGNANT MELANOMA", TUMOR BIOLOGY, vol. 9, no. 6, 1988, pages 301-306, XP008144612, ISSN: 1010-4283

## Description

The present invention concerns the use of melanoma biomarkers in medical and diagnostic field and method for the identification thereof. Particularly, the invention concerns a method for the identification of new disease biomarkers (e.g. cancer or diabetes mellitus) in undepleted serum by a novel multi-denaturation method and use of said cancer biomarkers for the diagnosis and treatment of cancer.

Early identification of serum markers may be extremely important in cancer patients. Several different approaches are currently followed in the attempt to identify markers relevant for early diagnosis or prognosis, and for therapeutic interventions. Cutaneous melanoma is a neoplasia characterized by high aggressiveness, early metastatic dissemination and poor prognosis once metastasized. Several circulating biomarkers in melanoma patients have been identified by different approaches, including proteomic analyses. For instance, S100B, C reactive protein (CRP) and lactate dehydrogenase (LDH) may help to determine the prognosis of melanoma patients⁶³. However, most of these and other serum biomarkers fail to predict the risk of tumor progression in early-stage melanoma patients (AJCC stage I to III) or the risk of tumor recurrence. Therefore, melanoma patients urgently need valid serological tools to reach diagnosis and predict prognosis, since presently the only biomarkers with a prognostic value in stage I-III are the histomorphological features of the primary tumor. Recently, serum amyloid A has been proposed as a prognostic marker in melanoma by MS-based proteomic profiling using hydrophobic C18 surfaced magnetic beads⁶⁴.

Proteins released in the bloodstream reflect the complex network of functions acting within the tissues. In plasma and serum it is possible to find: a) secretory proteins; b) immunoglobulins; c) hormones and peptides acting as long-distance signals; d) cytokines and short-distance signals, e.g. responsible for paracrine effects; e) products of cell or tissue damage as consequence of apoptotic or necrotic processes, including nucleic acids (most of the diagnostic markers used so far are within this class of molecules); f) aberrant secretory products, like the ones released under pathologic conditions; g) products of non-human origin, like proteins from bacteria, parasites or other micro-organisms, either pathogens or not.¹ Given the circulatory nature of the blood-stream, serum and plasma represent the most important sources of information regarding function of any peripheral district as well as the whole body under either normal or pathologic conditions. It is noteworthy that proteins and peptides released in blood-stream may be directly produced by the pathologic tissue or may represent a reaction of the microenvironment surrounding the pathologic tissue itself. Therefore, analysis of serum/plasmatic biomarkers may be very important for early diagnosis of cancer. Unfortunately, despite the relevant effort spent in the past years, sensitive and reproducible biomarkers for early diagnosis or pre-screening analysis are still lacking for many cancer types. In fact, serum proteome collected and characterized under standardized protocols contains a huge amount of molecules, but only in a limited part they are known as early markers of neoplastic diseases. It has been suggested that many potentially useful neoplastic markers are present in traces, below the detection level or still unrecognized². This makes serum biomarkers discovery a challenging field and an extremely hard task, despite the current improvement of analytical techniques. One difficulty comes from the huge concentration range existing between the most and the less abundant proteins in serum, encompassing at least 9 orders of magnitude. Moreover, the most represented proteins (e.g. albumin, haptoglobin, microglobulin, transferrin, immunoglubulins and a few others) account for more than 90% of the total serum proteome and often make very difficult to analyze the remaining 10%.^{3,4} To overcome such problem, depletion approaches are usually implemented aimed at removing the most abundant proteins in order to highlight the less abundant proteins.⁵⁻⁷ In fact, depletion approach often allows to detect proteins present in serum at concentrations lower than 10 ng/ml, i.e. proteins undetectable in a whole serum analysis.⁸ On the other hand, since abundant proteins often carry other smaller molecules, the "depletion approach" might remove (totally or in part) the carried signals, leading to relevant false negative detection or even alter the actual concentration detected in the blood samples.^{6,9} Further, depletion techniques may also significantly interfere with a delicate balance of serum protein/peptide solubility and folding, since a depleted serum does not completely reflect the physiological environment of blood proteins. Additionally, they may introduce an interpretation bias due to the carrier-peptides interactions behaviour and the loss of many signals related to protein-protein interaction.
An additional weakness of the existing protocols involving 2D-electrophoretic sera analysis, is related to the intrinsic features of serum. Individual differences may sum-up to the reproducibility issues of 2D analysis, making it difficult to compare sera derived from large groups of patients.¹⁰⁻¹³ To overcome the limitations of 2D electrophoresis, many Authors follow alternative approaches generally based on gel-free systems¹⁴⁻¹⁷, although in most cases the complexity of the developed strategies still do not make easy the analysis of large number of sera. On the other hand, mono-dimensional (1D) electrophoresis, while less informative than 2D-electrophoresis, allows the simultaneous separation of more sera from more patients, thus making it possible to highlight individual differences.

In light of the above, it is evident the need of new methods for the identification of cancer biomarkers which allow to overcome the limits of the so far adopted techniques. The same methods may be proposed for biomarker discovery of any other disease, like for instance diabetes mellitus.

The inventor of the present invention has now developed a novel procedure to analyze the whole serum without any depletion procedure, by investigating many different solubilisation/denaturation procedures consisting of different combinations of salts, detergents, temperature and osmotic factors, in order to improve the separation by 1D semi-preparative gradient gel electrophoresis. The present procedure analyzes serum protein patterns according to the ability to be differently denatured by different denaturation protocols, and, therefore, broken down into their components.

Particularly, according to the present invention, it has been demonstrated that the serum proteome susceptibility to denaturation (*denaturability*) may be a key feature, still underestimated since a new multi-denaturation protocol was able to discriminate serum proteins according to their *denaturability.* Sixty-nine different chemical/physical denaturation treatments were tested on pooled whole (undepleted) sera, and the 3 most discriminating (3 DENaturation Treatments, 3DEnT) were selected. Serum proteomes from mice and from patients carrying cutaneous early-stage melanoma were analyzed by semi-preparative gradient SDS-PAGE, were compared to healthy controls and differently expressed proteins were identified by mass-spectrometry. Seven and eight proteins were differentially expressed (p<0.05) in mice and human melanoma sera, respectively, compared to corresponding controls.

Some prior art references are known concerning tumor biomarkers. For instance, Jerome Solassol et al.⁷² (Jerome Solassol et al., Expert Review of Proteomics, vol. 6, no. 4, 1 August 2009, pages 341-343) disclose an in vitro method for diagnosing melanoma by detecting serum levels of biomarkers; Silver et al.⁷³ (Silver et al., British Journal of Cancer, Nature Publishing Group, vol. 41, no. 5, 1May 1980, pages 745-750) describe a study on the relationship of total serum sialic acid to sialylglycoprotein acute-phase reactants in malignant melanoma; Wojtukiewicz et al.⁷⁴ (Wojtukiewicz et al., Haemostasis, vol. 28, no. 1, January 1998, pages 7-13) disclose the profiles of plasma serpins in patients with advanced malignant melanoma, gastric cancer and breast cancer; Kwaan et al**.**⁷⁵ (Kwaan et al.Tumor Biology, vol. 9, no. 6, 1988, pages 301-306) describe a study on tissue plasminogen activator and inhibitors of fibrinolysis in malignant melanoma.

According to the method of the present invention, three proteins never before reported as differently expressed *in vivo* cancer models were found to be significantly modified both in mice and human melanoma: α2macroglobulin (down-regulated in cancer patients, validated by immunoblot analysis, p<0.001), Apo-E and Apo-1 (both up-regulated, p<0.02). These results indicate that: 1) serum proteome contains a large amount of information, still underestimated, linked to proteins folding; 2) investigating serum protein *denaturability* indicates novel strategies to discover biomarkers and molecular mechanisms of human diseases; 3) α2MG, Apo-E and Apo-1 may represent novel biomarkers for early melanoma diagnosis.

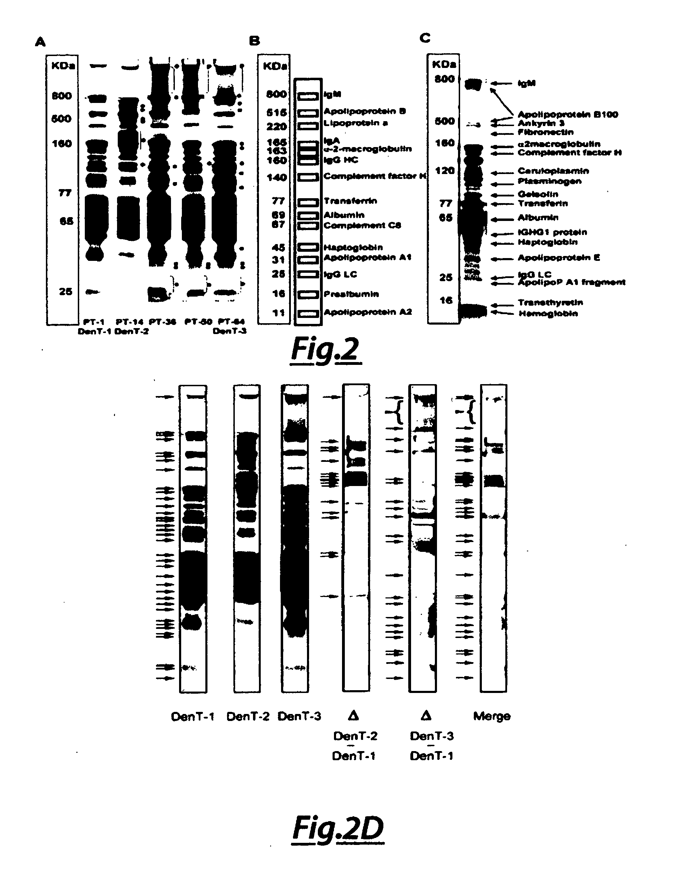

The whole serum proteome has been analyzed without any depletion step before the electrophoretic fractionation and evaluated simultaneously the sera of several patients and controls, thus minimizing the difficulties related to technical and individual reproducibility often occurring with 2D gels. Therefore, starting from not-depleted sera samples, the electrophoretic fractionation has been optimized by modifying a series of parameters, e.g. type of protein denaturation, range of polyacrylamide gradient, time length of electrophoretic separation. Optimal results were achieved using 16×18 cm slab gels with a continuous gradient of 2.4-15% acrylamide-bisacrylamide solution, followed by silver staining detection. It is noteworthy that, due to the different chemical-physical pre-treatments used in the various denaturation protocols, each protein band profile could be considered distinct from another one derived from the same sample undergoing a different denaturation protocol, since it reflects the presence of differently folded protein(s). To increase the protein band resolution, sera samples were previously subjected to many different pre-treatments based on the combination of chemical and physical different protein denaturing approaches, with the aim to increase the solvent accessibility to as many proteins and protein-complexes as possible. The three Denaturation Treatments (DenTs) showing the highest protein bands discrimination were chosen. Thereafter, each serum sample was analysed by three-denaturation treatments simultaneously applied (3DenT-SDS-PAGE). This multi-denaturation protocol, when performed on a significant number of samples and compared with control sera, allowed the comparison of proteins differentially detectable in cancer *vs* healthy sera. As result of this multi-denaturation approach, the differential detection of serum proteins may be due not only to the different expression level but also to different *"denaturability",* i.e. sensitivity to denaturation agents, of patient sera proteins when compared to healthy sera proteins. Different *denaturability* may be due, for instance, to different post-translational modifications, hydrophobic interactions, hydrogen bonds and other weak interactions with other proteins, including abundant carrier proteins, potentially related to specific pathological status. It is noteworthy that among the serum proteins separated by 3DenT-SDS-PAGE, the method of the invention is able to identify some proteins (e.g. ankyrin and IGHG1 protein) which usually are not readily detected by conventional SDS-PAGE of serum without any depletion or enrichment pre-treatment. This is likely due to the ability of the protocol of the invention to reveal protein bands otherwise "buried" in large complexes blocked into the stacking gel or not able to enter the gel itself. Therefore the protocol according to the present invention is very useful to improve the analysis of serum proteins or other complex mixtures of proteins. Further, differently from other techniques, due to the possibility to run simultaneously in the same gel several samples from different patients or controls, this protocol may be useful to study and identify novel biomarkers reproducibly modulated and, therefore, potentially useful for early detection of cancer in whole sera. The significant differences of protein pattern in serum samples pre-treated with DenT-1, compared to DenT-2 and DenT-3 protocols, indicate that 3DenT was likely able to disaggregate some protein complexes, and/or improve their solubilisation and electrophoretic separation (Fig. 2D). This indicates that serum proteome is much more complex than it is usually believed, since there is a significant body of information related to the *denaturability* of serum proteins that is usually neglected.

As shown in the reported stained gels of serum protein separated by 3DenT (Fig. 2, 3 and 4), it is possible that the same protein band is differently detectable in one lane when compared to another one, i.e. when subjected to a different denaturation protocol. This was observed in many cases and in a reproducible manner, suggesting that each protein band can be quantitatively and/or qualitatively different form each other, even though migrating at the same position, depending on the DenT used. This indicates that each protein band detected in each lane of our 3DenT analysis can be considered virtually different from any others. Therefore, 3DenT protocol allows to analyze, via further MS studies, at least 36+53+59=148 virtually different proteins from one undepleted serum sample. This number can be increased if the multi-denaturation protocol is carried out with more than 3 DenTs, see for instance Fig. 2A, where the pooled serum was denatured under 5 different protocols and more than 200 bands were discriminated.

The first, unexpected, result of the present study was therefore the finding that serum proteome contains a large body of information, which may be roughly identified by differential *denaturability,* grossly underestimated but potentially meaningful for new biomarker discovery.

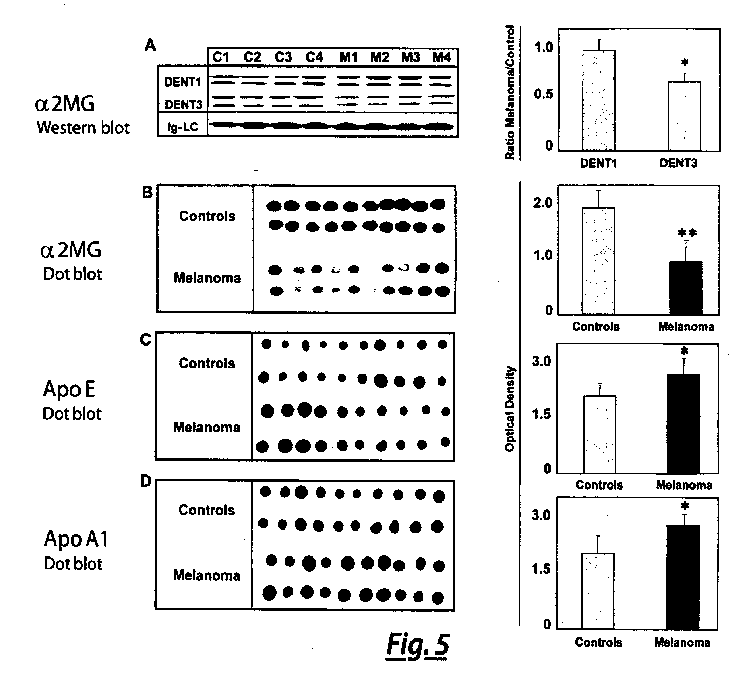

To verify such result, 3DenT protocol was therefore applied to a specific cancer model, i.e. sera from mice bearing cutaneous melanoma at early stage compared to sera from control mice and, subsequently, sera from early-non-metastatic melanoma patients compared to sera from healthy individuals. In either animal model and human cancer, three protein bands were found to be reproducibly and significantly (p <0.05) up- or down-regulated in melanoma serum proteome compared to controls, while other proteins resulted specifically modified in mouse only or human only cancer model (see Table 5A and 5B). One of the most significantly modified proteins was the α2-macroglobulin (α2MG), a potent protease inhibitor able to modulate diverse cellular processes, including cell adhesion, proliferation and migration, which are involved in cancer progression *in vitro.*⁶⁵⁻⁶⁷ Other significantly modified proteins in cancer vs control sera, in both murine and human cancer, were Apo-E and Apo-1. Therefore, as a consequence of the application of the new developed 3DenT protocol, a novel diagnostic protein signature for human melanoma has been discovered. A validation study was performed by immunological analyses with human sera from melanoma and controls subjects, using a commercial antibody raised against human α2MG, confirming that: a) the protein band differentially expressed in sera from cutaneous melanoma patients was human α2MG indeed; b) this protein was indeed down-regulated in sera from cutaneous melanoma patients when compared to healthy control sera. The differential expression of human α2MG in whole sera fractionated by 3DenT protocol demonstrated by Western Blot (WB) was significant (p<0.04) and confirmed by immunoblot (i.e. without electrophoretic fractionation) of whole sera from a larger number of patients with a very high statistically significant difference (p<0.0005). It is noteworthy that in WB analysis, the α2MG expression difference between pathological sera and controls was better evidenced in the DenT-3 treatment than in DenT-1 (Fig. 5A) confirming that different denaturation protocols may reveal different protein(s) pattern. Further, it is noteworthy that the protocol according to the present invention was developed to optimize the protein electrophoretic discrimination of whole serum proteome, i.e. without any depletion, but, of course, it is possible to apply this protocol to depleted serum samples or to any other complex mixtures of proteins. Altogether these results indicate that the different *denaturability* of proteins may be successfully used to identify proteins within complex protein mixtures which are slightly, but significantly, modified in pathological *vs* control conditions. A different sensitivity to denaturation of a protein complex may reflect and highlight structural modifications due to post-translational processes otherwise difficult to be evidenced by conventional mono- or bi-dimensional techniques. Thus, the described 3DenT protocol is able to increase the range of protein discrimination by SDS-PAGE revealing small but reproducibly significant differences in serum proteome.

The involvement of α2MG in aggressiveness of human cancer cells was hypothesized by *in vitro* studies with human prostate and breast cancer^{68,69} and in melanoma cells⁷⁰, but the present study represent the first *in vivo* evidence, from a murine cancer model confirmed in human patients, indicating that α2MG, Apo-1 and Apo-E may be used as novel biomarkers for cancer diagnosis. In summary, the results reported in the present study, starting from a new methodological approach, represent: a) the first evidence that serum proteome *denaturability* contains a large body of information, still underestimated, indicating a novel strategy to biomarkers discovery; b) the first *in vivo* evidence about a role of α2MG, Apo-E and Apo-1 as early diagnostic biomarkers in cutaneous melanoma patients.

Therefore, it is an object of the present invention a method for *in vitro* diagnosis of melanoma comprising or consisting of evaluating or measuring the expression in serum of all the following eight biomarkers: alpha-2 macroglobulin, transthyretin, Apo E, Apo A1, Apo B, ceruloplasmin, alpha-fetoprotein and albumin, wherein transthyretin, Apo E, Apo A1, Apo B are over-expressed in comparison to the expression thereof in a healthy subject, meanwhile alpha-2 macroglobulin, ceruloplasmin, alpha-fetoprotein and albumin are down-expressed in comparison to the expression thereof in a healthy subject.

The method according to the present invention can be carried out by means of antibodies against all the biomarkers as defined above. In addition, the present invention concerns a kit for *in vitro* diagnosis of melanoma comprising or consisting of antibodies against all the biomarkers as defined above.

The present invention now will be described by way of illustration and not limitation, according to preferred embodiments thereof, with particular reference to the enclosed drawings wherein:

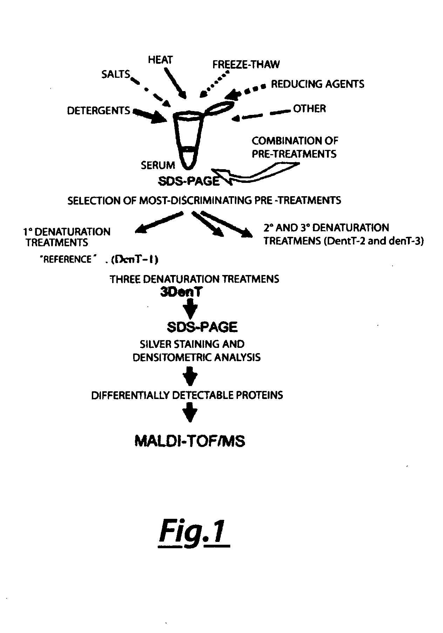

**Figure 1****:** The multi-denaturation protocol: schematic diagram of chemical-physical pre-treatments of mouse or human sera.
**Figure 2****: A:** Representative electrophoretic separation of human pooled sera derived from the most discriminating sample pre-treatments (defined in Table 1 as DenT-2 and DenT-3 treatments) compared to the control treatments (DenT-1). Samples were run on a manually poured gradient slab gel (2, 4 - 15%): 120 µg of proteins loaded per lane. Black asterisks indicate some of the protein bands undetectable in DenT-1 but more evident or newly represented in DenT-2 or DenT-3 pre-treatments. **B:** The serum protein pattern as described in literature and as obtained with 3DenTs protocol in a 16×18 cm gradient SDS-PAGE (2.5-15%) **(C),** by MALDI-TOF/MS analysis of cut and digested protein bands. **D:** Graphical representation of the improvement of protein band resolution and discrimination after DenT-2 or DenT-3 pre-treatments compared to the DenT-1. The protein pattern identified as Merge indicates the gain of protein detection and discrimination.

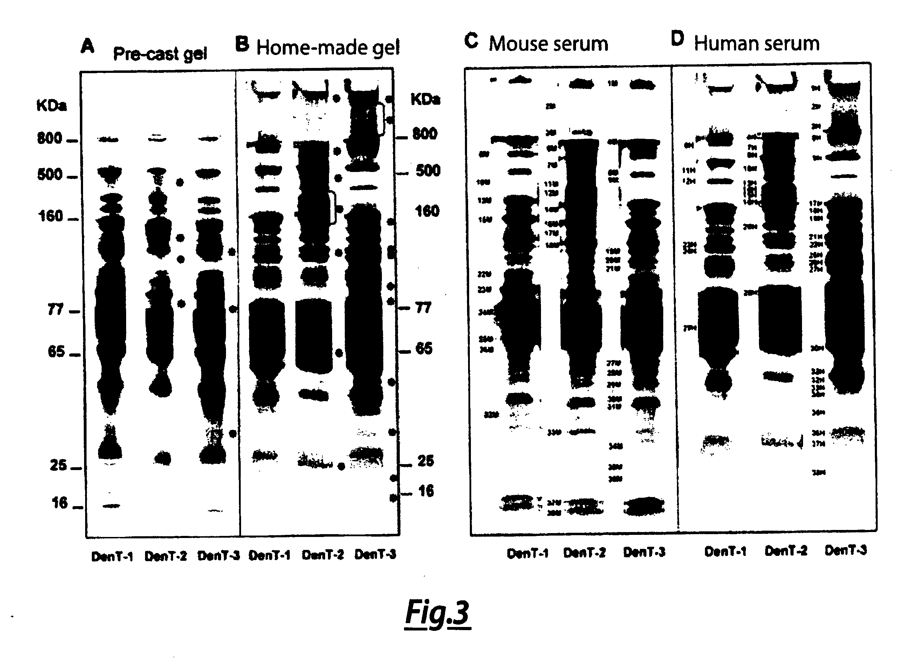

**Figure 3****: A** and **B:** 3DenT-SDS-PAGE using pre-cast and manually poured 8×8 cm gels, respectively. Representative electrophoretic human pooled sera patterns are shown, after application of 3DenT multi-denaturation protocol, run on pre-cast gradient gel (4-12%) and manually poured 2.4-15% gradient gel (in both cases 8×8 cm gels, 1 mm thickness). Equal amount of serum proteins (25 µg) were loaded onto both type of gel and asterisks indicate some of protein bands newly detectable in consequence of the pre-treatment compared with α pre-treatment (control). **C** and **D:** murine (M) and human (H) representative 3DenT electrophoretic patterns, respectively. The bands of interest are identified with a number and a letter, and their identification is reported in Tab. 5A and 5B.

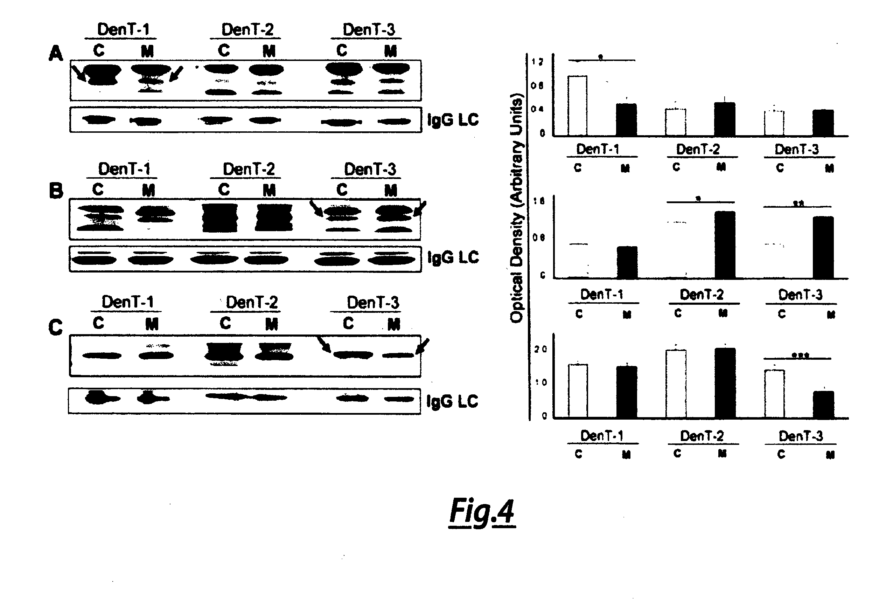

**Figure 4****:** Silver staining of three different bands whose expression resulted modulated in control (C) *vs* melanoma (M) mice sera. Statistical analysis was performed on groups of 6 mice per treatment. The reported bands, marked by arrows, are those found significantly differently expressed in cancer sera compared to controls, in at least 3 independent experiments. **A:** Band M21 (Complement factor B) is down-modulated in sera from melanoma affected mice, in DenT-1 condition, compared to the control (* = p<0.0476). **B:** Band M32 (Apolipoprotein E) is up-modulated in sera from melanoma affected mice compared to the control, both in DenT-2 (* =p<0.04083) and in DenT-3 condition (** = p<0.0056). C: Band M15 (alpha2Macroglobulin) is down-modulated in sera from melanoma affected mice in DenT-3 condition, compared to the control (*** = p<0.0074).
**Figure 5****:** Immunological validation of Alpha 2 Macroglobulin (α2MB) as diagnostic marker. **A:** Representative WB with anti-α2MB on human sera from 4 healthy and 4 melanoma affected individuals. The sera were pre-treated with the 3DenT protocol, fractionated on gradient manually poured gels and submitted to electro-blotting. In the figure only the DenT-1 and DenT-3conditions are shown. Each lane was loaded with 120 µg of serum proteins. Densitometric analysis (right panel), reports the ratio Melanoma/Ctrl expression for the α2MB band, revealed as a doublet, under this pre-treatment protocol (* = p<0.039). The same amount of protein was loaded per lane, as confirmed by comparing immunoglobulin light chain (Ig-LC) stained by Blue Coomassie. **B:** Immunoblot with anti-human α2MG on human sera from 10 healthy (Controls) and 10 melanoma affected individuals. The sera diluted 1:5 with PBS were spotted in duplicate on nitrocellulose membrane (50 µg of total proteins per spot, equal amount of loaded proteins checked by Bradford assay and Ponceau Red) and submitted to primary, then HRP-conjugated secondary antibodies incubation followed by chemiluminescence detection (** = p<0.001). Densitometric analysis (right panel) reports the comparative evaluation of α2MB expression. Data are reported as means ± SD. **C and D:** Immunoblot with anti-human Apo E and Apo A1, respectively, on human sera from 10 healthy (Controls) and 10 melanoma affected individuals. (* = p<0.001). Data are reported as means ± SD.

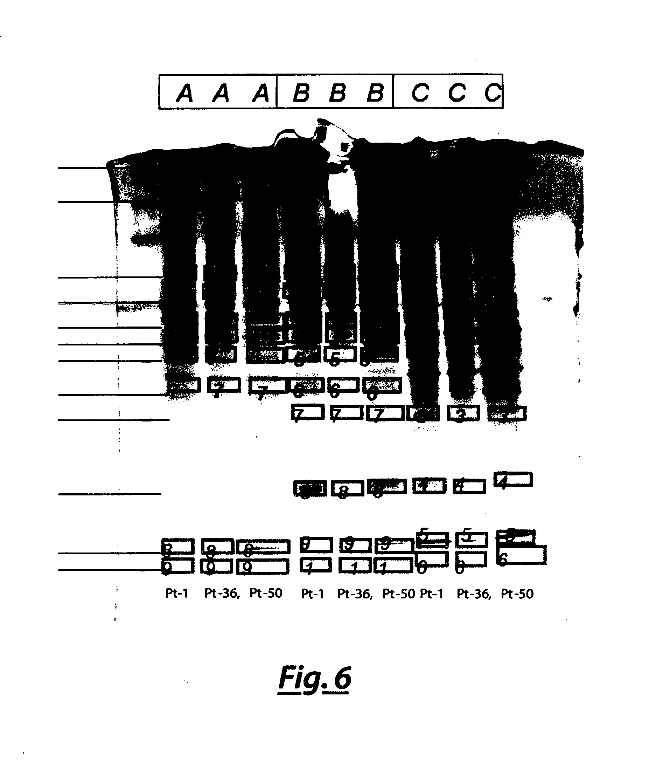

**Figure 6****:** TRIDENT analysis of 3 human melanoma cell lisates: A = human melanoma cell with low aggressiveness; B = human melanoma cell with intermediate aggressiveness; C= human melanoma cell with high aggressiveness. TRIDENT was performed using PT-1, PT-36 and PT-50 protocols.

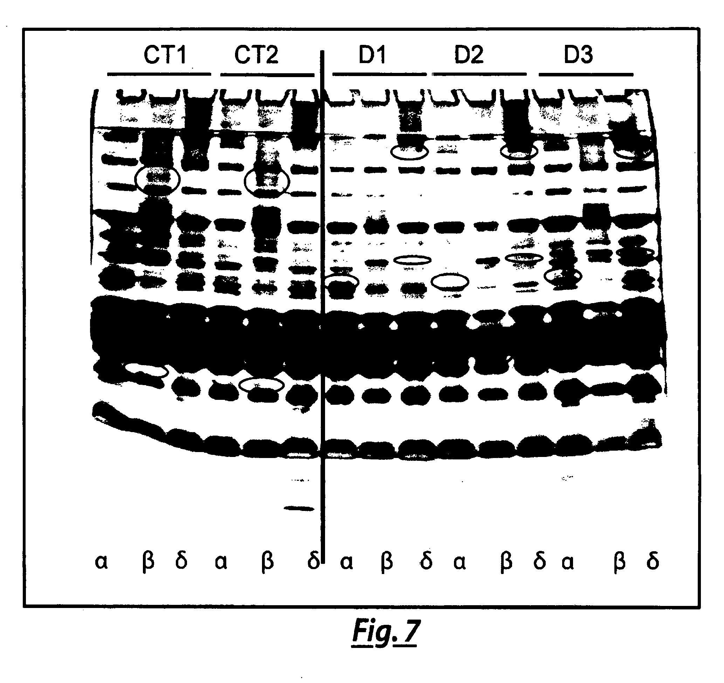

**Figure 7****:** TRIDENT analysis of Control (CT) and Diabetic (D) sera. α, β,δ correspond to Pt-1, Pt-14 and Pt-64. Protein bands detectable only (or better detected) in Control sera or in Diabetic sera are circled.

### Example 1: Multi-denaturation protocol for the identification of cancer biomarkers in undepleted serum

Melanoma has been extensively studied, and several potential diagnostic biomarkers have been identified, among them 3 (α2MG, Apo E and Apo A1) have been also validated by immunological methods. The identified diagnostic biomarkers may be useful to develop ELISA kits. Other cancer types (summarized in Additional Table) have been investigated and preliminary results indicate that TRIDENT protocol (Pt-1, Pt-14 and Pt-64) is also able to discriminate many differentially expressed proteins (not yet identified), potentially useful for diagnostic purposes (for example to develop ELISA kits).

### Materials and Methods

### Electrophoresis

Slab gels (2.4 -15% continuous acrylamide-bisacrylamide gradient vertical gels, manually poured into 16×18 cm, thickness 1.5 mm, 15 wells) were generated with a gradient maker (Model 385, Bio-Rad, Hercules California, US) and run with the SE 600 Ruby Apparatus (Hoefer, Inc. Holliston, MA), using fresh solutions. The final concentration of the gel reagents used were the following: acrylamide-bisacrylamide 3 - 0.08%, 125 mM Tris-HCl pH 8.8, 0.1% SDS (w/v), 0.1% ammonium persulphate (w/v), 0.07% TEMED (v/v) in the stacking gel and 600 mM Tris-HCl pH 6.8, 0.078% SDS (w/v), 0.045% ammonium persulphate (w/v), 0.047% TEMED (v/v) in the resolving gradient gel. To avoid any exogenous protein contamination, all procedures involving gel pouring, polymerization and handling were performed in a sterile class 2 cabinet. The sample buffer solution used was defined "high stringency sample buffer" (HSSB) containing, as final concentration, 44 mM Tris-HCl pH 6.8, 2% SDS (w/v), 10% Glycerol (v/v), 5% 2-β-mercaptoethanol (v/v) and 0.0125% Bromophenol Blue (w/v); it was prepared as 2× stock solution. The pre-loading denaturation, which samples were subjected to immediately before the electrophoretic run, was carried out as following: a 2x "high stringency sample buffer" (HSSB) was mixed to each sample in 1:1 ratio; then solution was heated for 7 minutes in a thermoblock pre-heated at 95 °C (Thermomixer Compact by Eppendorf, Hamburg, Germany) then immediately placed on ice (0-3 °C). Electrophoretic running conditions were: 60 min at 100 Volt constant followed by 220 min at 160 Volt constant (at 15 °C under thermostatic control). At the end of the run the whole gel, including the stacking portion, was handled. For some experiments, 25 µg of proteins of the same samples were loaded both into Precast Gradient Gels (NuPAGE Novex Bis-Tris, 4-12% acrylamide gradient gel, 8x8 cm, thickness 1 mm, 15 wells, from Invitrogen, Carlsbad, CA, US: running conditions were according to the manufacturer's instructions) and into manually poured gels of the same size (run in vertical electrophoretic chambers from Hoefer, Inc. Holliston, MA). The protein bands were detected in the gels by silver staining protocol as previously described by Shevchenko¹⁸, with the following modifications: time of soaking of the gel in 0.1% AgNO₃ was 30 min; developer solution was prepared in the dark, using sodium carbonate dissolved immediately before starting the staining procedure and fresh solution of DTT; all the steps were carried out at room temperature (20 °C); development was stopped by adding 1:1 volume of 10% citric acid.

Acrylamide, bisacrylamide and 2-β-mercaptoethanol were from ICN Biomedicals (Irvine, CA, US); AgNO₃ was from Merck Eurolab (Lutterworth, Leicestershire, UK). All other chemicals and reagents used, analytical grade, were from Sigma-Aldrich (St. Louis, Missouri, US).

### Densitometric analysis

Bands densitometry was carried out using the Bio-Rad Quantity One Software (Hercules, CA, US); the stained gels were scanned using a Bio-Rad ChemiDoc apparatus equipped with a high resolution digital camera and the photos were saved as file pictures (TIF format) to be further analyzed for densitometry by Bio-Rad Quantity One Software. Bands expression was evaluated as normalized optical density and only protein bands with a mean density significantly higher or lower (p<0.05) than corresponding controls were considered differentially expressed.

### Serum pre-treatments: the 3DenT Protocol

FBS (Fetal bovine Serum, from Sigma-Aldrich, US) aliquots and a pool of human sera (from 28 healthy individuals) were subjected to 69 different chemical or physical pre-treatments, before electrophoretic fractionation. The different 69 denaturation treatments have been described and summarized in Table 1 (see results). The first ones consisted of treatments with detergents, tested on serum at concentration of 1 or 2% (Nonidet 40, TritonX100, Tween 20, SDS), salts between 0.5 and 5 M (Sodium Chloride and Ammonium Bicarbonate) and reducing agents (2-βmercaptoethanol, at 5 or 10% final concentration or ((-)-1,4-Dithio-L-threitol, DTT, minimum 95%, Sigma Aldrich) 10 mM). Physical treatments were performed by exposing serum to temperature (e.g. 37 °C heating, 100 °C boiling or cycles of freeze and thaw for different time length and/or several times) and to ultrafiltration process using membrane filters with different cut-off (3, 10, 30 KDa, Centricon Centrifuge Filters from Millipore, Billerica, MA, US) or sonication. Each pre-treatment was tested alone or in combination with others (i.e. salts with temperature; detergent with temperature and ultrafiltration, and so on). Sodium chloride and ammonium bicarbonate were from Carlo Erba (Milan, IT). All other chemicals used were from Sigma Aldrich, US.

Human and bovine serum samples were analyzed by gradient SDS-PAGE and the two denaturation treatments able to resolve the highest number of serum protein bands, were selected, namely PT-14 and PT-64. These denaturation treatments were thereafter used in combination with a "control denaturation" treatment, i.e. the PT-1, in order to highlight any differential electrophoretic mobility of serum proteins due to the different ability to be denatured, *"denaturability".*

The electrophoretic profile of each serum was therefore obtained in 3 different lanes, corresponding to 3 different DenTs (Control/PT-1, PT-14 and PT-64) followed by gradient SDS-PAGE analysis. Such electrophoretic analysis was therefore called 3DenT-SDS-PAGE. Gels were stained by Coomassie R-250 or silver nitrate protocols. In either cases, the protein pattern obtained by PT-1 was different than those obtained by the others PTs, as a consequence of the serum proteome complexity. As expected, silver staining procedure was more sensitive than the Coomassie one, and revealed more protein bands and more significant differences among denaturation protocols. The silver stained gels were scanned and saved as TIF files and protein bands were densitometrically quantified. Protein normalization was achieved by loading equal amount of total proteins (Bradford assay) and checked by densitometric comparison with immunoglobulin light chain band.

### Human sera for electrophoretic studies

Human sera were obtained under full institutional review board approval and patient consent by vein puncture according to standard clinical protocols. For setting up experiments, a pool of human sera was generated by mixing 2 ml of each individual serum from 28 different healthy individuals, consisting of 14 male and 14 female, with age ranging between 25 and 65 years. Mean age of male and female individuals was 42.8 ± 13.2 and 44.9 ± 15.9, respectively. The hemato-clinical parameters (including glycemia, liver/kidney functionality tests, coagulation and lipidemic assays) for each individual were evaluated and only sera showing values within the physiological range were included.

For experiments with individual human sera, sera of 10 melanoma patients (5 males and 5 females), with reported diagnosis of cutaneous melanoma at early or non-metastatic stages (I-IIIB stages according to AJCC (NCCN Clinical Practice Guidelines in Oncology, Melanoma,V.2.2010, www.nccn.org) were compared to those from 10 healthy subjects (5 males and 5 females within the same age range).

### Cell culture

B16-F10 mouse melanoma cells were provided by ATCC (ATTC Number: CRL-6475). Cells were maintained in Dulbecco's modified Eagle's medium (DMEM, Gibco BRL, Paisley, UK) in the presence of 10% heat-inactivated calf serum (Gibco). B16-F10 cells were cultured in a CO₂ incubator (5% CO₂, 37 °C) in plastic culture flasks. Cells were detached by trypsin/EDTA harvesting and viable cells were identified by trypan blue exclusion and counted with a hemocytometer, afterward the number of cells to be injected in mice was resuspended in PBS (2×10⁵ cells/100 µl), as previously described⁷¹.

### Mass spectrometry analysis

In order to perform protein identification, bands differentially expressed were excised from silver-stained SDS-PAGE gels, reduced with DTT 10 mM ((-)-1,4-Dithio-L-threitol minimum 95%, Sigma Aldrich) for 45 minutes at 56 °C, alkylated with 55 mM Iodoacetamide (Sigma Ultra, Sigma Aldrich) at room temperature in the dark and digested with 0.1 mg/ml trypsin sequencing grade from bovine pancreas (Roche Applied Science, Monza, IT) in 25 mM ammonium bicarbonate (Sigma Aldrich) overnight at 37 °C. One microliter of the supernatant was loaded on a 96 wells plate (Applied Biosystem, Life Technologies Corporation, Carlsbad, CA) and analyzed by MALDI- time of flight mass spectrometer (TOF MS) (MALDI-TOF Voyager-DE STR, Applied Biosystems) after crystallization with α-cyano-4-hydroxycinnamic acid as matrix. When necessary, tryptic peptides were desalted by µC18 Zip Tip (Millipore). Spectra were analyzed by Data Explorer TM (Data Explorer Version 4.0.0.0 Copyright© 1997-2000, Applied Biosystem) and Moverz software (m/z - Knexus edition Copyright© 1998-2001 Proteometrics, LLC, New York, NY). Proteins were unambiguously identified by searching a comprehensive non-redundant protein database through MASCOT algorithm (Matrix Science, Peptide Mass Fingerprint). Only protein identification by mass fingerprinting with score >64 (i.e. p<0.05) were considered significant. Data mass accuracy was 50 ppm.

### Immunological analysis

In Western Blot (WB) analyses, human sera from healthy and melanoma individuals were treated with 3DenTs protocol and fractionated electrophoretically on gradient gel: 120 µg of serum proteins per lane and were loaded in the gel then blotted onto nitrocellulose membrane (Amersham Biosciences, Uppsala, Sweden). After blocking for 1 hour with 5% milk/PBS (low fatty acid milk powder from Sigma Aldrich solubilised in PBS without calcium and magnesium, PBS⁻⁻, pH 7.2), the membrane was incubated for 75 min with a goat primary antibody (diluted 1:1000 in 1.25% milk/PBS) against human a2MG (by Sigma). Then, the membrane was washed 3 times for 7 min in 0.1% Tween 20-PBS (T-PBS), incubated for 1 hour with secondary antibody (anti-goat HRP from Santa Cruz Biotechnology Inc., Santa Cruz, CA, diluted 1:10000 in 2% milk/PBS) and then washed again as before. Finally, the immunoreactions were visualized by ECL reagents (Amersham Biosciences) in dark room. All WB experiments were repeated at least 3 times. Protein loading was checked by Ponceau Red staining of membranes before blocking.

In dot-blot analyses, human sera from 10 healthy and 10 melanoma individuals were spotted onto nitrocellulose membrane (50 µg of proteins for each spot, repeated in duplicate). All melanoma patients were selected at early, non-metastatic stage. After blocking for 30 minutes with 5% milk/PBS, the membrane was incubated for 1 hour with rabbit anti-α2-macroglobulin antibody (1:1000 in 2% milk/PBS). Then, the membrane was washed 3 times with 0.1% T-PBS and incubated for 1 hour with secondary antibody as for the WB experiments. The signal was visualized with ECL method according to the manufacturer's instructions. All dot-blot experiments were repeated at least 3 times. Protein loading was checked and normalized by Ponceau Red staining of membranes before blocking.

### Animal experiments

An *in vivo* mouse primary melanoma growth assay was carried as previously reported with modifications, according to an accepted animal-study protocol.²⁰ Six adult male C57BL/6 mice (Jackson Laboratories, Bar Harbor, ME) were anesthetized by an intraperitoneal injection of 2.5% Avertin (Sigma Aldrich); they received by subcutaneous injection in the dorsal skinfold 2.5×10⁵ B16F10 cells dissolved in 100 µl of PBS. As controls, six mice were injected with PBS: the experiment was repeated 4 times, for a total of 24 mice injected with melanoma cells and 24 with PBS. Eight days after melanoma cell injection, 50-100 µl of blood were taken out by tail vein puncture from each control and melanoma injected mouse. Then blood samples were kept for 2 hours at room temperature to allow coagulation, and the serum was prepared by centrifugation. Three weeks after melanoma cell injections, tumours were removed surgically and weighted, fixed in formalin 4% for 48 hours and embedded in paraffin to confirm melanoma engraftment according to standard procedures. Procedures involving animals and their care were conducted in conformity with institutional guidelines in compliance with international laws and policies.

### Statistical analysis

The results were expressed as the mean ± standard error of the mean. Student's two tails t-test was carried out and P values lower than 0.05 were considered significant.

### Abbreviations

α2MG, α2-macroglobulin; 3DEnT, 3 DENaturation Treatments; DB, dot blot; DenT, Denaturation treatment; DTT, 1,4-Dithio-L-threitol; DMEM, Dulbecco's modified Eagle's medium; MS, mass spectrometry; MW, molecular weight; PAGE, polyacrylamide gel electrophoresis; WB, western blot.

### Results

### Human pool sera electrophoretic analysis

A preliminary study was first carried out to optimize the SDS-PAGE analysis of serum proteins starting from a pool of human sera from 28 healthy individuals (see Methods) to be used in the setting up methodological study. Serum was used as whole, i.e. without any depletion step. Nine and six different chemical and physical pre-treatments, respectively, and other 9 pre-treatments deriving from their combination, were tested on the pooled sera and the 3 DENaturation Treatments (3DenT) showing higher discrimination power and sensitivity were then selected (see Fig.1). The complete list of DenTs analyzed is reported in Table 1 and a schematic diagram showing the whole procedure is summarized in Fig. 1.

Table 1 shows the characteristics of the pre-treatments (PT, physical, chemical and combinations of them) tested on human and bovine sera.

**Table 1**

| **Name** | **Type of human / bovine serum pre-treatments** | **Description of serum denaturing pre-treatment** |
|---|---|---|
| **PT-1** | **Reference pre-treatment** | Dilution 1:1 with bi-distilled water. |
| PT-2 | **high salt pre-treatments** | Dilution 1:1 with solution of Sodium Chloride to reach a final concentration between 0.5 and 5 M (0.5 M =PT-2, 1 M =PT-3, 2.5 M =PT-4 and 5 M =PT-5). |
| PT-3 | | |
| PT-4 | | |
| PT-5 | | |
| PT-6 | **high salt pre-treatments** | Dilution 1:1 with solution of ammonium bicarbonate to reach a final concentration between 0.5 and 5 M (0.5 M =PT-6, 1 M =PT-7, 2.5 M =PT-8 and 5 M =PT-9). |
| PT-7 | | |
| PT-8 | | |
| PT-9 | | |
| PT-10 | **physical pre-treatments** | Dilution 1:1 with PBS followed |
| PT-11 | | by heating at 37 °C for 1 (PT-10) or 3 days (PT-11) |
| PT-12 | **physical pre-treatments** | Dilution 1:1 with PBS followed by 3 (PT-12) or 10 cycles of freeze and thaw (PT-13). |
| PT-13 | | |
| PT-14 | **physical pre-treatments** | Dilution 1:1 with PBS followed by boiling at 100°C for a period of time varying between 2.5 and 10 minutes, centrifuge at 10.000 g for 15 min, room temperature, with subsequent careful pellet recovery (2.5 min = PT-14, 5 min = PT-15, 10 min = PT-16). |
| PT-15 | | |
| PT-16 | | |
| PT-17 | **physical pre-treatments** | Dilution 1:1 with PBS followed by boiling at 100°C for different times between 2.5 and 10 minutes with subsequent supernatant recovery (2.5 min = PT-17, 5 min = PT-18, 10 min = PT-19). |
| PT-18 | | |
| PT-19 | | |
| PT-20 | **physical pre-treatments** | Dilution 1:1 with PBS followed by ultra-filtration with 3, 10, 30 KDa cut off membranes (3 KDa = PT-20, 10 KDa = PT-21, 30 KDa = PT-22). |
| PT-21 | | |
| PT-22 | | |
| PT-23 | **detergent pre-treatments** | Dilution 1:1 with detergent (Nonidet P40) to reach 1, 1.5 or 2% final concentration (1% = PT-23, 1.5% = PT-24, 2% = PT-25). |
| PT-24 | | |
| PT-25 | | |
| PT-26 | **detergent pre-treatments** | Dilution 1:1 with detergent (Triton X100) to reach 1, 1.5 or 2% final concentration (1% = PT-26, 1.5% = PT-27, 2% = PT-28). |
| PT-27 | | |
| PT-28 | | |
| PT-29 | **detergent pre-treatments** | Dilution 1:1 with detergent (Tween 20) to reach 1, 1.5 or 2% final concentration (1% = PT-29, 1.5% = PT-30, 2% = PT-31). |
| PT-30 | | |
| PT-31 | | |
| PT-32 | **chemical pre-treatments** | Dilution 1:1 with HSSB with 2-β mercaptoethanol (2-βMSH) at 1, |
| PT-33 | | |
| PT-34 | | 2.5, 5, 10% (v/v) final concentration (1% = PT-32, 2.5%=PT-33, 5%=PT-34, 10%=T-35). |
| PT-35 | | |
| PT-36 | **chemical pre-treatment** | Dilution 1:1 with HSSB without 2-βMSH, followed by boiling at 100°C for 2.5 min. |
| PT-37 | **chemical/physical pre-treatments** | Dilution 1:1 with Sodium chloride at 1 M final concentration followed by boiling at 100°C for different period of time (1 min =PT-37, 2.5 min = PT-38, 5 min = PT-39, 10 min = PT-40). |
| PT-38 | | |
| PT-39 | | |
| PT-40 | | |
| PT-41 | **chemical/physical pre-treatments** | Dilution 1:1 with Sodium chloride at different concentration (0, 0.5, 1, 2 M) followed by ultra-filtration (no NaCl = PT-41, 0.5M = PT-42, 1M = PT-43, 2M =PT-44). |
| PT-42 | | |
| PT-43 | | |
| PT-44 | | |
| PT-45 | **high temperature pre-treatments** | Dilution 1:1 with HSSB (without 2-βMSH) followed by boiling at 100°C for 2.5 = PT- 45 or 5 minutes = PT-46 (pellet recovery). |
| PT-46 | | |
| PT-47 | **high temperature pre-treatments** | Dilution 1:1 with HSSB (5% 2-βMSH), followed by boiling at 100°C for 2.5 = PT-47, 5 = PT-48 or 10 minutes = PT 49 (pellet recovery). |
| PT-48 | | |
| PT-49 | | |
| PT-50 | **strong reducing / detergent and high temperature pre-treatments** | Dilution 1:1 with HSSB (10% 2-βMSH), followed by boiling at 100°C for 2.5 = PT-50, 5 = PT-51 or 10 minutes = PT 52 (pellet recovery). |
| PT-51 | | |
| PT-52 | | |
| PT-53 | **chemical and physical pre-treatments** | Dilution 1:1 with HSSB (5% 2-βMSH for 5, 10 or 20 min at 37°C) followed by 0.45 µm |
| PT-54 | | |
| PT-55 | | |
| | | filtration to remove large aggregates: 5 min = PT-53, 10 min = PT-54, 20 min = PT-55. |
| PT-56 | **chemical and physical pre-treatments** | Dilution 1:1 with HSSB (10% 2-βMSH for 5, 10 or 20 min at 37°C) followed by 0.45 µm filtration: 5 min = PT-56, 10 min = PT-57, 20 min = PT-58. |
| PT-57 | | |
| PT-58 | | |
| PT-59 | **chemical and physical pre-treatment** | Dilution 1:1 with HSSB (without 2-βMSH) followed by filtration |
| PT-60 | **short chemical and physical pre-treatments** | dilution 1:1 with sonication buffer (SB) followed by sonication for 10 minutes by using a sonicating bath at room temperature, then followed by dilution 1:1 with HSSB (5% 2-βMSH) and boiling at 100°C for 2.5 = PT-60 or 5 minutes = PT-61. |
| PT-61 | | |
| PT-62 | **longer chemical and stronger physical pre-treatments** | dilution 1:1 with sonication buffer (SB) followed by sonication for 30 minutes by using a sonicating bath at room temperature, then followed by dilution 1:1 with HSSB (5% 2-βMSH) and boiling at 100°C for 2.5 =PT-62 or 5 minutes = PT-63. |
| PT-63 | | |
| PT-64 | **longer chemical and stronger physical pre-treatments** | dilution 1:1 with sonication buffer (SB) followed by sonication for 60 minutes by using a sonicating bath at room temperature,τ then followed by dilution 1:1 with HSSB (5% 2-βMSH) and boiling at 100°C for 2.5 =PT-64 or 5 minutes = PT-65. |
| PT-65 | | |
| PT-66 | **longer chemical and stronger physical pre-treatments** | Serum sonication for 90 minutes by using a sonicating bath at room temperature; then dilution |
| PT-67 | | |
| | | 1:1 with HSSB (5% 2-βMSH) followed by boiling at 100°C for 5 = PT-66 or 10 minutes = PT-67 |
| PT-68 | **longer chemical and stronger physical pre-treatments** | Serum sonication for 90 minutes at 38 °C; then dilution 1:1 with HSSB (5% 2-βMSH) followed by boiling at 100°C for 5 = PT-68 or 10 minutes = PT-69 |
| PT-69 | | |

The pre-treatment 1 (PT-1) condition corresponding to 1:1 dilution with distilled water followed by denaturation according to standard Laemmli procedure, was thereafter identified as "DenT-1". Two additional DenTs, corresponding to those showing the highest protein bands discrimination power, were selected, namely the PT-14 and PT-64 ones, as summarized in Fig. 1 and Table 1, and identified as DenT-2 and DenT-3. Therefore each serum was denatured according to 3 different DenT and thereafter reported as 3DenT-SDS-PAGE followed by silver-staining protein detection. Data reported in Fig.2 indicate that a markedly different serum protein pattern is detectable by gradient SDS-PAGE in lanes undergoing different DenTs, indicating that the serum proteome may be differently electrophoresed by SDS-PAGE when different denaturation treatments are applied. In fact, in several cases (see asterisks in Fig. 2A) a specific DenT revealed a different electrophoretic serum pattern in Table 2 summarizes the number of total protein bands discriminated through 3 different DenTs on the same serum and demonstrates that 3DenT protocol may significantly improve the SDS-PAGE separation of whole undepleted serum proteins.

Table 2 shows human pool serum bands resolution in the gradient vertical slab gel 2.4 - 15%, 16×18 cm, and effects of the serum pre-treatments (3DenT) on the gel bands discrimination. It is noteworthy that the total numbers of bands detectable under DenT-2 (53±1) or DenT-3 (59±2) pre-treatment reflect the gain of bands compared to the DenT-1 (see also Fig. 2D). Therefore, the 3DenT allows to analyze about 76 different protein bands for each serum sample, a number significantly higher than that representing the protein bands detected by the Laemmli treatment alone (DenT-1, 36 bands).

**Table 2**

| **Pre-treatment** | DenT-1 | DenT-2 | DenT-3 | DenT-1+DenT-2+DenT-3 |
|---|---|---|---|---|
| **Total protein bands detectable** | 36±1 | 53±1 | 59±2 | 36+53+59 = **148** |
| **Newly detected bands (vs DenT-1)** | | 17 | 23 | |
| **Total number of different bands to analyze** | 36±1 | 36+17=53 | 36+23=59 | 36+17+23 = **76** |

In fact, 17 additional bands are found with DenT-2 and 23 additional bands are found with DenT-3, as compared to DenT-1. In order to compare the pattern of separated proteins with the known serum proteome, the proteins separated by DenT-1 were excised and subjected to MALDI-TOF/MS identification. Fig. 2B-C show the protein bands reproducibly identified by MALDI-TOF/MS compared to the serum protein pattern known from the literature. ^{3, 21-25} Therefore, while the single denaturation treatment (DenT-1) was able to discriminate 36 different serum protein bands (black arrows in Fig. 2D), the 3DenT protocol detected an additional set of at least 40 protein bands in the same serum sample (blue and red arrows in Fig. 2D, 16x18 cm gel). The protein sensitivity of the detection method was estimated to be lower than 5 ng/band. It is noteworthy that 3DenT-SDS-PAGE was able to reveal some proteins in human serum, namely H2 MHC-I antigen, the IGHG1 protein, Q10 α-chain and the ankyrin isoform 3, which are undetectable or extremely difficult to be identified in serum fractionated with conventional procedure followed by MALDI-TOF/MS analysis.^{33,62} In Table 3 the serum/plasma levels of some proteins separated with 3DenT-SDS-PAGE and identified by MALDI-TOF/MS are listed as reported in literature^{26,48,52}.

Table 3 shows the sensitivity of the 3DenT-SDS PAGE compared to bibliographic references. Some of human and murine serum proteins identified with 3DenT-SDS-PAGE followed by MALDI-TOF/MS are reported. For each identified protein, the following information is reported: annotations, i.e. theoretical MW and NCBI protein accession number, %C = percentage coverage, #P = number of unique peptides identified, and the serum/plasma concentration levels known by literature.

**Table 3**

| **Protein name** | **Source** | **%C** | **#P** | **Annotations** | **Type of condition** | **[ ] in serum or plasma from bibliography** | **Methods of isolation and detection from bibliography** |
|---|---|---|---|---|---|---|---|
| *Alpha-2 macroglobulin* | Human and Mouse | 40 | 44 | MW: 163278 Da (human) | DenT-1, DenT-2 and DenT-3 treatments | Plasma and serum protein (8-10% of the total serum proteome).^{26,27} Range: 1-6g/L | Radial immunodiffusion; laser nephelometry.²⁸ 2D (gel dependent and not) coupled with MS. |
| | | | | Accession: P0123 (human) | | | |
| | | | | MW: 165881 Da (mouse) | | | |
| | | | | Accession: Q61838 (mouse) | | | |
| *Ankyrin 3* | Human | 10 | 19 | MW: 479853 Da Accession: Q5VXD5 | DenT-2 treatment | Erythrocyte integral membrane protein (cytoskeletal adaptor protein).²⁹ | ELISA³⁰; capillary gel electrophoresis³¹; immunoprecipitation and immunoblotting ³². |
| | | | | | | | High abundant proteins depletion plus conventional separation techniques combined with tandem mass spectrometry (MS/MS) |
| *H2-MHC-I antigen*, *Q10 chain* | Human and Mouse | 29 | 9 | MW: 37251 Da Accession: P01898 | DenT-3 treatment | Membrane protein also secreted in blood. | Glycosylation large scale analysis and mass spectrometry.³³ |
| | | | | MHC class I family. Some different haplotypes. | | | |
| *Apolipoprotein E* | Human and Mouse | 46 | 20 | MW: 36154 Da (human) Accession: P02649 (human) | DenT-1, DenT-2 and DenT-3 treatments | Plasma and serum protein Range in plasma: 35-50 mg/L | Immunoturbidimetry³⁵; Capillary electrophoresis (SDS-CGE) ³⁶; radioimmunoassay ³⁷; immunoblotting and isoelettrofocusing.³⁸ |
| | | | | MW: 35848 Da (mouse) Accession: Q4FK40 (mouse) | | Range in serum: 30-40mg/L³⁴ | |
| | | | | Multiallelic | | | |
| | | | | gene (E1, E2, E3, E4) | | | |
| *C4B5* | Human | 35 | 10 | MW: 47581 Da Accession: Q6U2L9 | DenT-3 treatment | Plasma and serum protein | Single radial immunodiffusion method³⁹; immunological methods; high voltage agarose gel lectrophoresis plus immunofixation plus aemolytic overlay method⁴⁰; 2D (gel dependent and not) coupled with MS. |
| | | | | Rare allotype (1/100) | | Range of C4 group in serum: 0.2-0.5 g/L.³⁹ | |
| *Gelsolin* | Human and Mouse | 37 | 30 | MW: 85698 Da (human) Accession: Q5T0I2 (human) | DenT-1 and DenT-3 treatments | Cytoskeletal protein. Normally present in plasma. Lower | Quantitative Western Blotting⁴²; mmunoblotting⁴³ 2-DIGE plus MS ⁴⁴; Immunonephelometry⁴⁵; ELISA assay⁴⁶; functional assays^{47,48} |
| | | | | MW: 85942 Da (mouse) Accession: P13020 | | concentration in serum. Range in plasma: 0.1-0.3 mg/ml⁴¹ | |
| *Transthyretin (prealbumin)* | Human and Mouse | 72 | 7 | MW: 15887 Da (human) Accession: P02766 (human) | DenT-1 and DenT-3 treatments | Plasma and serum protein Range in serum: 0.1-0.4 g/L⁴⁹ | 2DE plus MS⁵⁰; 1DE plus MS ⁵¹ Immunoprecipitation analysis⁵², multidimensional chromatography followed by MS/MS⁵³; electroimmunodiffusion⁵⁴; immunoblotting. |
| | | | | MW: 15776 Da (mouse) Accession: Q5M9K1 (mouse) | | | |
| *IGHG1 protein* | Human | 28 | 8 | MW: 51716 Da Accession: Q6PJA4 | DenT-3 treatment | Plasma and serum protein | Immunofluorescence, immunoblotting and immunohistochemistry⁵⁵ |
| | | | | Multiple | | | |
| | | | | allotypes (z,x,a,f) | | | |
| *HP protein (Haptoglobin)* | Human | 25 | 7 | MW: 31382 Da Accession: Q6NSB4 | DenT-3 treatment | Plasma and serum glycoprotein Range in serum: 0.3-2.5 g/L⁵⁶ | 2-DIGE followed by MS or ELISA ⁵⁷; immunoblotting or 2DE plus MS⁵⁶; photometric method and capillary zone electrophoresis method (CZE) ^{58,59}; multilectin affinity chromatography + nanoLC-ESIMS/MS⁶⁰; immunohistochemistry.⁶¹ |
| | | | | Polymorphic gene; phenotypes: Hp 1-1, Hp 2-1, and Hp 2-2 | | | |

### Selection of polyacrilamide gradient and comparison of manually poured gradient -SDS-PAGE gels vs pre-cast gradient -SDS-PAGE gels

In order to select the most effective acrylamide gradient, to investigate the resolution power of our electrophoretic protocols and to compare it to others, we fractionated human pooled sera proteins, pre-treated according to the 3DenT protocol, on precast gradient gels and on manually generated gradient gels of the same size, with several different gradients (2.4-12%, 2.4-15%, 4-12%, 4-15%). In these experiments, the most sensitive electrophoretic separation was achieved by using manually generated 2.4-15% acrylamide-bisacrylamide (30:0.8) continuous gradient gel. A representative SDS-PAGE of human serum is reported and the comparison between pre-cast and manually poured gels is shown (Fig. 3A and 3B). The resolution power, evaluated as the number of protein bands detectable on the gel, is summarized in Table 4, showing that the manually generated 2.4-15% gradient gel, although requiring a more complex manipulation, was able to discriminate a significantly higher number of protein bands in the same serum sample.

Table 4 shows human pooled sera bands resolution and comparison between 3DenT-manually poured SDS-PAGE and 3DenT-pre-cast gradient gel SDS-PAGE, in both cases run onto 8×8 cm gels. Data are reported as mean ±SD.

**Table 4**

| | **DenT-1** | **DenT-2** | **DenT-3** |
|---|---|---|---|
| **Total protein bands detectable in manually poured gradient gel** | 34±1 | 39±1 | 36±1 |
| **Total protein bands detectable in gradient pre-cast gel** | 27±1 | 20±1 | 28±1 |
| **"Bands gain" with manual compared to precast gel** | **7±1** | **19±1** | **8±1** |

### 3DenT-SDS-PAGE of murine and human sera: a differential study to compare cancer and healthy sera

Mouse melanoma model study: the 3DenT-SDS-PAGE electrophoretic method was then applied to the study of murine sera from 6 mice bearing cutaneous melanoma vs 6 healthy mice, in 4 independent experiments (Fig. 3C). Each protein band from DenT-1, DenT-2 and DenT-3 protocols was represented as mean of 6 densitometric values. The melanoma sera bands whose densitometric mean values were significantly up- or down-regulated with a significant statistical difference from the control sera (p<0.05) were considered of interest, therefore they were cut and subsequently analyzed by MALDI-TOF/MS. According to such procedure, at least 9 protein bands, with molecular masses ranging between 150 and 18 KDa, showed highly significant difference of expression. Other bands were, less significantly but reproducibly, modulated. Eight and two protein bands were found to be up- or down-regulated, respectively, in melanoma *vs* control mice (Table 5A).

Table 5A shows differentially expressed bands by 3DenT-SDS-PAGE in murine sera from cancer animal compared to the healthy controls. P value means the significance between densitometry of control (Ctrl) bands vs melanoma bands (Mel).

**Table 5A**

| **Mouse Band** | **p value** | **State change** | **Prot ID** | **AC #** |
|---|---|---|---|---|
| **15M** | 0.0074 | Up-regulated in Ctrl | α2-MacroGlobulin | Q61838 |
| **21M** | 0.0476 | Up-regulated in Mel | Complement Factor B | Q3UEG8 |
| **22M** | 0.0036 | Up-regulated in Mel | Gelsolin | P13020 |
| **23M** | 0.0167 | Up-regulated in Ctrl | Transferrin | Q921I1 |
| **24M** | 0.0354 | Up-regulated in Mel | Albumin | P07724 |
| **27M** | 0.0093 | Up-regulated in Mel | Albumin | P07724 |
| | 0.0111 | | | |
| **28M** | 0.0051 | Up-regulated in Mel | C3 protein | Q80XP1 |
| | 0.0194 | | Apo A-VI | Q91XF |
| **29M** | 0.03568 | Up-regulated in Mel | MHC I Antigen H2Q10 | P01898 |
| **32M** | 0.0408 | Up-regulated in Mel | Apo E | Q4FK40 |
| | 0.0056 | | | P08226 |
| **35M** | 0.0295 | Up-regulated in Mel | Apo A1 | Q00623 |

Human melanoma model study. To verify the ability to discriminate protein bands in human sera from cancer patients, the same approach was carried out in 10 melanoma patients compared to 10 control healthy sera (Fig. 3D). MALDI-TOF/MS mass spectrometry analysis was then carried out in order to identify protein(s) differentially electrophoresed and detected (Table 5B).

Tab. 5B shows differentially expressed bands by 3DenT-SDS-PAGE in human sera from cancer patients compared to the healthy controls. P value means the significance between densitometry of control bands *vs* melanoma bands.

**Table 5B**

| **Human Band** | **p value** | **State change** | **Prot ID** | **AC #** |
|---|---|---|---|---|
| **4H** | 0.0047 | Up-regulated in Mel | Apo B | P04114 |
| **16H** | 0.0255 | Up-regulated in Ctrl | Albumin | Q56G89 |
| **18H** | 0.0286 | Up-regulated in Ctrl | α2-MacroGlobulin | P01023 |
| **19H** | 0.04306 | Up-regulated in Ctrl | Ceruloplasmin | Q1L857 |
| **20H** | 0.0320 | Up-regulated in Ctrl | α2-MacroGlobulin | P01023 |
| **27H** | 0.0506 | Up-regulated in Ctrl | α-Fetoprotein | P02771 |
| **28H** | 0.0630 | Up-regulated in Ctrl | α-Fetoprotein | P02771 |
| | | | Albumin | Q56G89 |
| **31H** | 0.0164 | Up-regulated in Mel | Apo E | P02649 |
| **34H** | 0.0096 | Up-regulated in Mel | Apo A1 | P02647 |
| | 0.0119 | | | |
| | 0.0477 | | | |
| **36H** | 0.05199 | Up-regulated in Mel | Transthyretin | P02766 |

Most interestingly, α2-macroglobulin (α2MG) and two lipoproteins (Apo E and Apo A1) resulted to be reproducibly down-modulated (α2MG) and up-regulated (Apo E and Apo A1) both in mice and in human cancer sera when compared to corresponding control sera.

To highlight the differences detected between Controls and Melanoma sera, the electrophoretic 3DenT separation of some bands from mice sera, namely Band M21 (Complement factor B), Band M32 (Apolipoprotein E) and Band M15 (alpha2Macroglobulin), has been shown in Fig. 4A, B and C, as measured by densitometric analysis (right panels).

### Immunological validation of the identified potential markers

To validate the efficiency and sensitivity of the 3DenT protocol, we confirmed the down-modulation of human α2-macroglobulin (α2MG), Apo-E and Apo A1 in human melanoma sera by two independent immunological methods, Western Blot (WB) and Dot Blot (DB) analyses, using an anti-human α2MG polyclonal antibody and anti-human Apo E and anti-human Apo A1 antibodies. As shown in Fig. 5, western blot and dot blot techniques clearly evidenced the down-modulation of serum α2MG in the pathological condition, as measured by densitometric analysis (right panels). Thus, we may conclude that sera of melanoma patients contain significantly lower levels of α2MG. As far as Apo E and Apo A1, dot blot confirmed that both proteins are up-regulated in melanoma sera (see Fig. 5).

It is noteworthy that the above described TRIDENT method was applied successfully to identify novel diagnostic biomarkers of cutaneous melanoma, allowing to identify a number of novel potentially useful diagnostic biomarkers. In addition, TRIDENT protocol, using Pt-1, Pt-14 and Pt-64 as denaturation protocols, has been successfully applied to undepleted sera from patients suffering from other cancer types, showing the ability to discriminate many protein bands differently expressed from healthy sera. Namely, TRIDENT analysis of cancer sera, compared to healthy controls, was carried out also on sera of patients suffering from the following cancer types. For each cancer type, at least 6 patients and 6 healthy controls have been analyzed:
- Breast cancer **(Diff.Prot.Bands >10)**
- Colon cancer **(Diff.Prot.Bands >9)**
- Lung cancers (NSCLC) **(Diff.Prot.Bands >13)**
- Prostate cancer **(Diff.Prot.Bands >14)**
- Ovary cancer **(Diff.Prot.Bands >12)**

In all these types of cancer, the TRIDENT analysis, followed by SDS-PAGE and silver staining protocol, showed that sera from cancer sera contain protein bands differentially expressed (up- or down-modulated) when compared to healthy control sera. The 3 protocols used were: PT-1, PT-14 and PT-64. The identification of the proteins present in these differentially expressed bands is under investigation by MALDI-TOF/MS and LC-MS/MS analyses. In parenthesis, the number of protein bands found differentially expressed between cancer and healthy sera (Diff.Prot.Bands) is reported. Only protein bands whose densitometric quantification was significantly (p<0.05) modified *vs* healthy controls have been taken into account. Each protein band may contain at least 10-20 different proteins, therefore we can assume that ms identification will reveal hundreds of proteins potentially useful as diagnostic biomarkers for any of the above mentioned cancer types.

### Example 2: Multi-denaturation protocol for the identification of cancer biomarkers in cell lysates

The multi-denaturation protocol of example 1 has been carried out on melanoma cells extracts from cells with different aggressiveness. For TRIDENT analysis of cell lysates, a complex protein mixture with composition and chemical behaviours completely different from those belonging to serum and plasma, the tests carried out have evidenced that the best combination of pre-treatments was PT-1, PT-36 and PT-50. TRIDENT protocol (Pt-1, Pt-36 and Pt-50) was successfully applied to melanoma cell lysates, showing the ability to differentiate the protein patterns of human melanoma cells with different aggressiveness (See figure 6). Particularly, TRIDENT analysis has been applied to 3 human melanoma cell lisates: A = human melanoma cell with low aggressiveness; B = human melanoma cell with intermediate aggressiveness; C = human melanoma cell with high aggressiveness. This type of analysis, followed by protein identification by MS, allows to identify molecular signals potentially responsible for the human melanoma cell aggressiveness, e.g. biomarkers for prognostic purposes. These proteins could represent new interesting aggressiveness predictors (prognostic biomarkers) to be potentially applied to develop novel antibodies or kits suitable for prognostic purposes, for example for histologic studies on bioptic specimens, to assign aggressiveness score to cancer specimens.

### Example 3: Multi-denaturation protocol for the identification of biomarkers of disease in undepleted sera from diabetic patients

TRIDENT protocol, using Pt-1, Pt-14 and Pt-64 as denaturation protocols, has been successfully applied to undepleted sera from patients suffering from diabetes mellitus, compared to healthy normoglycemic subjects. Also in this case, the protocol was able to discriminate proteins differentially expressed between diabetic and control sera (figure 7). These differentially expressed proteins, not yet identified, may be useful to develop antibodies or kits suitable for diagnostic or prognostic purposes, e.g. ELISA kits for diagnosis of diabetes, or for categorization of patients with different risk to develop serious complications of diabetes (prognostic purposes).

### References

1. Doolittle, R. F., Fibrinogen and Fibrin, in the Plasma Proteins: Structure, Function and Genetic Control., F.W., ed. Academic Press, 3rd Edition, N.Y. 1975, 2, 109-156.
2. Liotta, L. A.; Ferrari, M.; Petricoin, E., Clinical proteomics: written in blood. Nature 2003, 425 (6961), 905.
3. Anderson, N. L.; Anderson, N. G., The human plasma proteome: history, character, and diagnostic prospects. Mol Cell Proteomics 2002, 1 (11), 845-67.
4. Geho, D. H.; Liotta, L. A.; Petricoin, E. F.; Zhao, W.; Araujo, R. P., The amplified peptidome: the new treasure chest of candidate biomarkers. Curr Opin Chem Biol 2006, 10 (1), 50-5. Wang, Y. Y.; Cheng, P.; Chan, D. W., A simple affinity spin tube filter method for removing high-abundant common proteins or enriching low-abundant biomarkers for serum proteomic analysis. Proteomics 2003, 3 (3), 243-8.
6. Pieper, R.; Su, Q.; Gatlin, C. L.; Huang, S. T.; Anderson, N. L.; Steiner, S., Multi-component immunoaffinity subtraction chromatography: an innovative step towards a comprehensive survey of the human plasma proteome. Proteomics 2003, 3 (4), 422-32.
7. Rothemund, D. L.; Locke, V. L.; Liew, A.; Thomas, T. M.; Wasinger, V.; Rylatt, D. B., Depletion of the highly abundant protein albumin from human plasma using the Gradiflow. Proteomics 2003, 3 (3), 279-87.
8. Pieper, R.; Gatlin, C. L.; Makusky, A. J.; Russo, P. S.; Schatz, C. R.; Miller, S. S.; Su, Q.; McGrath, A. M.; Estock, M. A.; Parmar, P. P.; Zhao, M.; Huang, S. T.; Zhou, J.; Wang, F.; Esquer-Blasco, R.; Anderson, N. L.; Taylor, J.; Steiner, S., The human serum proteome: display of nearly 3700 chromatographically separated protein spots on two-dimensional electrophoresis gels and identification of 325 distinct proteins. Proteomics 2003, 3 (7), 1345-64.
9. Righetti, P. G.; Boschetti, E.; Lomas, L.; Citterio, A., Protein Equalizer Technology : the quest for a "democratic proteome". Proteomics 2006, 6 (14), 3980-92.
10. Almeras, L.; Briolant, S.; Orlandi-Pradines, E.; Fontaine, A.; Henry, M.; Bogreau, H.; Pradines, B.; Rogier, C.; Fusai, T., [Proteomic analysis and parasitosis: principles and applications]. Med Trop (Mars) 2007, 67 (2), 188-96.
11. Witzmann, F. A.; Richardson, M. R., Two-dimensional gels for toxicological drug discovery applications. Expert Opin Drug Metab Toxicol 2006, 2 (1), 103-11.
12. Lopez, J. L., Two-dimensional electrophoresis in proteome expression analysis. J Chromatogr B Analyt Technol Biomed Life Sci 2007, 849 (1-2), 190-202.
13. Fuchs, D.; Winkelmann, I.; Johnson, I. T.; Mariman, E.; Wenzel, U.; Daniel, H., Proteomics in nutrition research: principles, technologies and applications. Br J Nutr 2005, 94 (3), 302-14.
14. Lohaus, C.; Nolte, A.; Bluggel, M.; Scheer, C.; Klose, J.; Gobom, J.; Schuler, A.; Wiebringhaus, T.; Meyer, H. E.; Marcus, K., Multidimensional chromatography: a powerful tool for the analysis of membrane proteins in mouse brain. J Proteome Res 2007, 6 (1), 105-13.
15. Steel, L. F.; Haab, B. B.; Hanash, S. M., Methods of comparative proteomic profiling for disease diagnostics. J Chromatogr B Analyt Technol Biomed Life Sci 2005, 815 (1-2), 275-84.
16. Romijn, E. P.; Krijgsveld, J.; Heck, A. J., Recent liquid chromatographic-(tandem) mass spectrometric applications in proteomics. J Chromatogr A 2003, 1000 (1-2), 589-608.
17. Camerini, S.; Polci, M. L.; Restuccia, U.; Usuelli, V.; Malgaroli, A.; Bachi, A., A novel approach to identify proteins modified by nitric oxide: the HIS-TAG switch method. J Proteome Res 2007, 6 (8), 3224-31.
18. Shevchenko, A.; Wilm, M.; Vorm, O.; Mann, M., Mass spectrometric sequencing of proteins silver-stained polyacrylamide gels. Anal Chem 1996, 68 (5), 850-8.
19. Facchiano, A.; Russo, K.; Facchiano, A. M.; De Marchis, F.; Facchiano, F.; Ribatti, D.; Aguzzi, M. S.; Capogrossi, M. C., Identification of a novel domain of fibroblast growth factor 2 controlling its angiogenic properties. J Biol Chem 2003, 278 (10), 8751-60.
20. Marcellini, M.; De Luca, N.; Riccioni, T.; Ciucci, A.; Orecchia, A.; Lacal, P. M.; Ruffini, F.; Pesce, M.; Cianfarani, F.; Zambruno, G.; Orlandi, A.; Failla, C. M., Increased melanoma growth and metastasis spreading in mice overexpressing placenta growth factor. Am J Pathol 2006, 169 (2), 643-54.
21. Ping, P.; Vondriska, T. M.; Creighton, C. J.; Gandhi, T. K.; Yang, Z.; Menon, R.; Kwon, M. S.; Cho, S. Y.; Drwal, G.; Kellmann, M.; Peri, S.; Suresh, S.; Gronborg, M.; Molina, H.; Chaerkady, R.; Rekha, B.; Shet, A. S.; Gerszten, R. E.; Wu, H.; Raftery, M.; Wasinger, V.; Schulz-Knappe, P.; Hanash, S. M.; Paik, Y. K.; Hancock, W. S.; States, D. J.; Omenn, G. S.; Pandey, A., A functional annotation of subproteomes in human plasma. Proteomics 2005, 5 (13), 3506-19.
22. Chromy, B. A.; Gonzales, A. D.; Perkins, J.; Choi, M. W.; Corzett, M. H.; Chang, B. C.; Corzett, C. H.; McCutchen-Maloney, S. L., Proteomic analysis of human serum by two-dimensional differential gel electrophoresis after depletion of high-abundant proteins. J Proteome Res 2004, 3 (6), 1120-7.
23. Anderson, N. L.; Polanski, M.; Pieper, R.; Gatlin, T.; Tirumalai, R. S.; Conrads, T. P.; Veenstra, T. D.; Adkins, J. N.; Pounds, J. G.; Fagan, R.; Lobley, A., The human plasma proteome: a nonredundant list developed by combination of four separate sources. Mol Cell Proteomics 2004, 3 (4), 311-26.
24. Liu, T.; Qian, W. J.; Gritsenko, M. A.; Xiao, W.; Moldawer, L. L.; Kaushal, A.; Monroe, M. E.; Varnum, S. M.; Moore, R. J.; Purvine, S. O.; Maier, R. V.; Davis, R. W.; Tompkins, R. G.; Camp, D. G., 2nd; Smith, R. D., High dynamic range characterization of the trauma patient plasma proteome. Mol Cell Proteomics 2006, 5 (10), 1899-913.
25. Chen, Y. Y.; Lin, S. Y.; Yeh, Y. Y.; Hsiao, H. H.; Wu, C. Y.; Chen, S. T.; Wang, A. H., A modified protein precipitation procedure for efficient removal of albumin from serum. Electrophoresis 2005, 26 (11), 2117-27.
26. Lisowska-Myjak, B.; Pachecka, J.; Kaczynska, B.; Miszkurka, G.; Kadziela, K., Serum protease inhibitor concentrations and total antitrypsin activity in diabetic and non-diabetic children during adolescence. Acta Diabetol 2006, 43 (4), 88-92.
27. Arinola, G.; Arowojolu, A.; Bamgboye, A.; Akinwale, A.; Adeniyi, A., Serum concentrations of immunoglobulins and acute phase proteins in Nigerian women with preeclampsia. Reprod Biol 2006, 6 (3), 265-74.
28. Molvarec, A.; Prohaszka, Z.; Nagy, B.; Kalabay, L.; Szalay, J.; Fust, G.; Karadi, I.; Rigo, J., Jr., Association of increased serum heat shock protein 70 and C-reactive protein concentrations and decreased serum alpha(2)-HS glycoprotein concentration with the syndrome of hemolysis, elevated liver enzymes, and low platelet count. J Reprod Immunol 2007, 73 (2), 172-9.
29. Hayashi, T.; Su, T. P., Regulating ankyrin dynamics: Roles of sigma-1 receptors. Proc Natl Acad Sci U S A 2001, 98 (2), 491-6.
30. Pekrun, A.; Eber, S. W.; Kuhlmey, A.; Schroter, W., Combined ankyrin and spectrin deficiency in hereditary spherocytosis. Ann Hematol 1993, 67 (2), 89-93.
31. Lin, C.; Cotton, F.; Boutique, C.; Dhermy, D.; Vertongen, F.; Gulbis, B., Capillary gel electrophoresis: separation of major erythrocyte membrane proteins. J Chromatogr B Biomed Sci Appl 2000, 742 (2), 411-9.
32. Chang, T. L.; Cubillos, F. F.; Kakhniashvili, D. G.; Goodman, S. R., Ankyrin is a target of spectrin's E2/E3 ubiquitin-conjugating/ligating activity. Cell Mol Biol (Noisy-le-grand) 2004, 50 (1), 59-66.
33. Bernhard, O. K.; Kapp, E. A.; Simpson, R. J., Enhanced analysis of the mouse plasma proteome using cysteine-containing tryptic glycopeptides. J Proteome Res 2007, 6 (3), 987-95.
34. Shielde, F.; De Bacquer, D.; Vincent-Viry, M.; Beisiegel, U.; Ehnholm, C.; Evans, A.; Kafatos, A.; Martins, C.M.; Sans, S.; Sass, C.; Visvikis, S.; De Backer, G.; Siest, G., Apolipoprotein E Serum concentration and Polymorphism in six European Countries: the ApoEurope Project. Atherosclerosis 2000, 152(2), 475-88.
35. Vincent-Viry, M.; Schiele, F.; Gueguen, R.; Bohnet, K.; Visvikis, S.; Siest, G., Biological variations and genetic reference values for apolipoprotein E serum concentrations: results from the STANISLAS cohort study. Clin Chem 1998, 44 (5), 957-65.
36. Schlenck, A.; Schiele, F.; Barbier, A.; Shuvaev, V. V.; Visvikis, S.; Siest, G., Capillary electrophoretic analysis of recombinant human apolipoprotein E. Calibration mode of a protein reference material. J Chromatogr A 1999, 853 (1-2), 237-41.
37. Havel, R. J.; Kotite, L.; Vigne, J. L.; Kane, J. P.; Tun, P.; Phillips, N.; Chen, G. C., Radioimmunoassay of human arginine-rich apolipoprotein, apoprotein E. Concentration in blood plasma and lipoproteins as affected by apoprotein E-3 deficiency. J Clin In vest 1980, 66 (6), 1351-62.
38. Haddy, N.; De Bacquer, D.; Chemaly, M. M.; Maurice, M.; Ehnholm, C.; Evans, A.; Sans, S.; Do Carmo Martins, M.; De Backer, G.; Siest, G.; Visvikis, S., The importance of plasma apolipoprotein E concentration in addition to its common polymorphism on inter-individual variation in lipid levels: results from Apo Europe. Eur J Hum Genet 2002, 10 (12), 841-50.
39. Yang, Y.; Chung, E. K.; Zhou, B.; Blanchong, C. A.; Yu, C. Y.; Fust, G.; Kovacs, M.; Vatay, A.; Szalai, C.; Karadi, I.; Varga, L., Diversity in intrinsic strengths of the human complement system: serum C4 protein concentrations correlate with C4 gene size and polygenic variations, hemolytic activities, and body mass index. J Immunol 2003, 171 (5), 2734-45.
40. Park, M. H.; Oh, M. D.; Song, Y. W.; Pai, H. J.; Takeuchi, F.; Tokunaga, K.; Hong, G. H.; Park, K. S., Association of complement alleles C4AQ0 and C4B5 with rheumatoid arthritis in Koreans. Ann Rheum Dis 1996, 55 (10), 776-8.
41. Dahl, B.; Schiodt, F. V.; Ott, P.; Wians, F.; Lee, W. M.; Balko, J.; O'Keefe, G. E., Plasma concentration of Gc-globulin is associated with organ dysfunction and sepsis after injury. Crit Care Med 2003, 31 (1), 152-6.
42. Mounzer, K. C.; Moncure, M.; Smith, Y. R.; Dinubile, M. J., Relationship of admission plasma gelsolin levels to clinical outcomes in patients after major trauma. Am J Respir Crit Care Med 1999, 160 (5 Pt 1), 1673-81.
43. DiNubile, M. J.; Stossel, T. P.; Ljunghusen, O. C.; Ferrara, J. L.; Antin, J. H., Prognostic implications of declining plasma gelsolin levels after allogeneic stem cell transplantation. Blood 2002, 100 (13), 4367-71.
44. Okano, T.; Kondo, T.; Kakisaka, T.; Fujii, K.; Yamada, M.; Kato, H.; Nishimura, T.; Gemma, A.; Kudoh, S.; Hirohashi, S., Plasma proteomics of lung cancer by a linkage of multi-dimensional liquid chromatography and two-dimensional difference gel electrophoresis. Proteomics 2006, 6 (13), 3938-48.
45. Dahl, B.; Schiodt, F. V.; Ott, P.; Gvozdenovic, R. ; Yin, H. L.; Lee, W. M., Plasma gelsolin is reduced in trauma patients. Shock 1999, 12 (2), 102-4.
46. Ito, H.; Kambe, H.; Kimura, Y.; Nakamura, H.; Hayashi, E.; Kishimoto, T.; Kishimoto, S.; Yamamoto, H., Depression of plasma gelsolin level during acute liver injury. Gastroenterology 1992, 102 (5), 1686-92.
47. Smith, D. B.; Janmey, P. A.; Herbert, T. J.; Lind, S. E., Quantitative measurement of plasma gelsolin and its incorporation into fibrin clots. J Lab Clin Med 1987, 110 (2), 189-95.
48. Lee, P. S.; Drager, L. R.; Stossel, T. P.; Moore, F. D.; Rogers, S. O., Relationship of plasma gelsolin levels to outcomes in critically ill surgical patients. Ann Surg 2006, 243 (3), 399-403.
49. Cano, N. J., Metabolism and clinical interest of serum transthyretin (prealbumin) in dialysis patients. Clin Chem Lab Med 2002, 40 (12), 1313-9.
50. He, Q. Y.; Lau, G. K.; Zhou, Y.; Yuen, S. T.; Lin, M. C.; Kung, H. F.; Chiu, J. F., Serum biomarkers of hepatitis B virus infected liver inflammation: a proteomic study. Proteomics 2003, 3 (5), 666-74.
51. Sundsten, T.; Eberhardson, M.; Goransson, M.; Bergsten, P., The use of proteomics in identifying differentially expressed serum proteins in humans with type 2 diabetes. Proteome Sci 2006, 4, 22.
52. Chertow, G. M.; Ackert, K.; Lew, N. L.; Lazarus, J. M.; Lowrie, E. G., Prealbumin is as important as albumin in the nutritional assessment of hemodialysis patients. Kidney Int 2000, 58 (6), 2512-7.
53. Zhang, R.; Barker, L.; Pinchev, D.; Marshall, J.; Rasamoelisolo, M.; Smith, C.; Kupchak, P.; Kireeva, I.; Ingratta, L.; Jackowski, G., Mining biomarkers in human sera using proteomic tools. Proteomics 2004, 4 (1), 244-56.
54. Bleiberg-Daniel, F.; Le Moullac, B.; Maire, J. C.; Wade, S., Failure of tryptophan deficiency to reduce specifically serum levels of transthyretin or albumin in rats. J Nutr 1990, 120 (12), 1610-6.
55. Chen, Z.; Gu, J., Immunoglobulin G expression in carcinomas and cancer cell lines. Faseb J 2007, 21 (11), 2931-8.
56. Li, S. Q.; Yun, J.; Xue, F. B.; Bai, C. Q.; Yang, S. G.; Que, H. P.; Zhao, X.; Wu, Z.; Wang, Y.; Liu, S. J., Comparative proteome analysis of serum from acute pulmonary embolism rat model for biomarker discovery. J Proteome Res 2007, 6 (1), 150-9.
57. Hoagland, L. F. t.; Campa, M. J.; Gottlin, E. B.; Herndon, J. E., 2nd; Patz, E. F., Jr., Haptoglobin and posttranslational glycan-modified derivatives as serum biomarkers for the diagnosis of nonsmall cell lung cancer. Cancer 2007, 110 (10), 2260-8.
58. Wuyts, B.; Delanghe, J. R.; Kasvosve, I.; Langlois, M. R.; De Buyzere, M. L.; Janssens, J., A new method for fast haptoglobin phenotyping and hemoglobin binding capacity calculation based on capillary zone electrophoresis. Clin Chem Lab Med 2000, 38 (8), 715-20.
59. Pirlot, A.; Janssens, J.; Skinner, G.; Godeau, J. M., Quantitative determination of haptoglobin (HAP) in human and bovine sera by capillary zone electrophoresis (CZE). Vet Res 1999, 30 (5), 483-93.
60. Heo, S. H.; Lee, S. J.; Ryoo, H. M.; Park, J. Y.; Cho, J. Y., Identification of putative serum glycoprotein biomarkers for human lung adenocarcinoma by multilectin affinity chromatography and LC-MS/MS. Proteomics 2007, 7 (23), 4292-302.
61. Juan, H. F.; Chen, J. H.; Hsu, W. T.; Huang, S. C.; Chen, S. T.; Yi-Chung Lin, J.; Chang, Y. W.; Chiang, C. Y.; Wen, L. L.; Chan, D. C.; Liu, Y. C.; Chen, Y. J., Identification of tumor-associated plasma biomarkers using proteomic techniques: from mouse to human. Proteomics 2004, 4 (9), 2766-75.
62. Chan, K.C.; Lucas, D.A.; Hise, D.; Schaefer, C.F.; Xiao, Z.; Janini, G.M.; Buetow, K.H.; Issaq, H.J.; Veenstra, T.D.; Conrads T.P. Analysis of the human serum proteome. Clinical Proteomics 2004, 1(2), 101-225.
63. Mouawad, R.; Spano, J.P.; Khayat, D. Old and new serological biomarkers in melanoma: where we are in 2009. Melanoma Res 2010, 20(2):67-76.
64. Findeisen, P.; Zapatka, M.; Peccerella, T.; Matzk, H.; Neumaier, M.; Schadendorf, D.; Ugurel, S. Serum amyloid A as a prognostic marker in melanoma identified by proteomic profiling. J Clin Oncol. 2009, 27(13), 2199-208.
65. Bizik, J.; Lizonova, A.; Grofova, M.; Matoska, J.; Dore, J. F.; Bertrand, S.; Blasko, M.; Vaheri, A., Clonal variation in the production of tumor-associated alpha 2-macroglobulin in a malignant human melanoma and association with growth stimulation. Cancer Res 1989, 49 (4), 983-90.
66. Matoska, J.; Wahlstrom, T.; Vaheri, A.; Bizik, J.; Grofova, M., Tumor-associated alpha-2-macroglobulin in human melanomas. Int J Cancer 1988, 41 (3), 359-63.
67. Lizonova, A.; Bizik, J.; Grofova, M.; Vaheri, A., Coexpression of tumor-associated alpha 2-macroglobulin and growth factors in human melanoma cell lines. J Cell Biochem 1990, 43 (4), 315-25.
68. Kancha, R. K.; Stearns, M. E.; Hussain, M. M., Decreased expression of the low density lipoprotein receptor-related protein/alpha 2-macroglobulin receptor in invasive cell clones derived from human prostate and breast tumor cells. Oncol Res 1994, 6 (8), 365-72.
69. Harthun, N. L.; Weaver, A. M.; Brinckerhoff, L. H.; Deacon, D. H.; Gonias, S. L.; Slingluff, C. L., Jr., Activated alpha 2-macroglobulin reverses the immunosuppressive activity in human breast cancer cell-conditioned medium by selectively neutralizing transforming growth factor-beta in the presence of interleukin-2. J Immunother 1998, 21 (2), 85-94.
70. Katerinaki, E.; Evans, G. S.; Lorigan, P. C.; MacNeil, S., TNF-alpha increases human melanoma cell invasion and migration in vitro: the role of proteolytic enzymes. Br J Cancer 2003, 89 (6), 1123-9.
71. Faraone, D.; Aguzzi, M.S.; Toietta, G.; Facchiano, A.M.; Facchiano, F.; Magenta, A.; Martelli, F.; Truffa, S.; Cesareo, E.; Ribatti, D.; Capogrossi, M.C.; Facchiano, A. Platelet-derived growth factor-receptor alpha strongly inhibits melanoma growth in vitro and in vivo. Neoplasia 2009, 11(8),732-42.
72. Jerome Solassol et al., Expert Review of Proteomics, vol. 6, no. 4, 1 August 2009, pages 341-343).
73. Silver et al., British Journal of Cancer, Nature Publishing Group, vol. 41, no. 5, 1 May 1980, pages 745-750.
74. Wojtukiewicz et al., Haemostasis, vol. 28, no. 1, January 1998, pages 7-13.
75. Kwaan et al.Tumor Biology, vol. 9, no. 6, 1988, pages 301-306.

## Claims

1. Method for *in vitro* diagnosis of melanoma comprising or consisting of evaluating the expression in serum of all the following eight biomarkers: alpha-2 macroglobulin, transthyretin, Apo E, Apo A1, Apo B, ceruloplasmin, alpha-fetoprotein and albumin wherein transthyretin, Apo E, Apo A1, Apo B are over-expressed in comparison to the expression thereof in a healthy subject, meanwhile alpha-2 macroglobulin, ceruloplasmin, alpha-fetoprotein and albumin are down-expressed in comparison to the expression thereof in a healthy subject.

2. Method according to claim 1, carried out by means of antibodies against all the biomarkers as defined in claim 1.

3. Kit for *in vitro* diagnosis of melanoma comprising or consisting of antibodies against all the biomarkers as defined in claim 1.

## Patentansprüche

1. Verfahren zur in-vitro-Diagnose von Melanomen, umfassend die oder bestehend aus der Auswertung der Expression aller folgenden acht Biomarker: Alpha-2-Macroglobulin, Transthyretin, Apo E, Apo A1, Apo B, Ceruloplasmin, Alpha-Fetoprotein und Albumin, in Serum, wobei Transthyretin, Apo E, Apo A1, Apo B im Vergleich zu deren Expression in einem gesunden Individuum überexprimiert sind, während Alpha-2-Macroglobulin, Ceruloplasmin, Alpha-Fetoprotein und Albumin im Vergleich zu deren Expression in einem gesunden Individuum unterexprimiert sind.

2. Verfahren nach Anspruch 1, welches mit Hilfe von Antikörpern gegen alle Biomarker wie in Anspruch 1 definiert durchgeführt wird.

3. Kit zur in-vitro-Diagnose von Melanomen, umfassend oder bestehend aus Antikörper(n) gegen alle Biomarker wie in Anspruch 1 definiert.

## Revendications

1. Procédé de diagnostic *in vitro* d'un mélanome, comprenant ou consistant en l'évaluation de l'expression dans du sérum des huit biomarqueurs suivants : alpha-2 macroglobuline, transthyrétine, Apo E, Apo A1, Apo B, céruloplasmine, alpha-foetoprotéine et albumine, dans lequel la transthyrétine, l'Apo E, l'Apo A1, l'Apo B sont surexprimées par comparaison avec l'expression de celles-ci chez un sujet sain, alors que l'alpha-2 macroglobuline, la céruloplasmine, l'alpha-foetoprotéine et l'albumine sont sous-exprimées par comparaison avec l'expression de celles-ci chez un sujet sain.

2. Procédé selon la revendication 1, effectué au moyen d'anticorps dirigés contre tous les biomarqueurs tels que définis dans la revendication 1.

3. Kit de diagnostic *in vitro* d'un mélanome, comprenant des ou consistant en anticorps dirigés contre tous les biomarqueurs tels que définis dans la revendication 1.
